(19) Europäisches Patentamt / European Patent Office / Office européen des brevets

(11) **EP 4 088 742 A2**

(12) **EUROPEAN PATENT APPLICATION**
published in accordance with Art. 153(4) EPC

(43) Date of publication:
**16.11.2022 Bulletin 2022/46**

(21) Application number: **21738122.7**

(22) Date of filing: **06.01.2021**

(51) International Patent Classification (IPC):
*A61K 47/54* (2017.01)   *A61K 47/64* (2017.01)
*A61K 31/7088* (2006.01)   *A61K 48/00* (2006.01)
*A61K 9/00* (2006.01)   *A61P 27/02* (2006.01)

(52) Cooperative Patent Classification (CPC):
**A61K 47/54**

(86) International application number:
**PCT/KR2021/000102**

(87) International publication number:
**WO 2021/141368 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2020 KR 20200001256**
**05.01.2021 KR 20210000730**

(71) Applicant: **Seasun Therapeutics**
**Daejeon 34015 (KR)**

(72) Inventors:
• **LEE, Dong In**
  **Daejeon 34675 (KR)**

• **KIM, Hye Joo**
  **Daejeon 34080 (KR)**
• **YU, Ji-Yeon**
  **Cheongju-si Chungcheongbuk-do 28193 (KR)**
• **KANG, Yusun**
  **Gimhae-si Gyeongsangnam-do 50898 (KR)**
• **PARK, Hee Kyung**
  **Daejeon 34034 (KR)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **COMPOSITION FOR PREVENTING OR TREATING MACULAR DEGENERATION, CONTAINING CELL PERMEABLE NUCLEIC ACID COMPLEX AS ACTIVE INGREDIENT**

(57) The present invention relates to a composition for preventing or treating macular degeneration, containing a cell permeable nucleic acid complex as an active ingredient and, more specifically, to a pharmaceutical composition for preventing, ameliorating, or treating macular degeneration comprising, a cell-permeable nucleic acid complex containing a bioactive nucleic acid targeting an NLRP3 gene; and a carrier peptide nucleic acid which are complementarily bound to said bioactive nucleic acid, as an active ingredient. The cell permeable nucleic acid complex, in which the bioactive nucleic acid targeting the NLRP3 gene and the carrier peptide nucleic acid are complementarily bound to each other, according to the present invention, has high cell permeability and thus inhibits the expression of NLRP3 without direct injection, and thereby is useful for preventing, ameliorating, or treating macular degeneration.

FIG. 3

**Description**

[Technical Field]

The present invention relates to a composition for preventing or treating macular degeneration containing a cell-permeable nucleic acid complex as an active ingredient, and more particularly to a pharmaceutical composition for preventing, ameliorating, or treating macular degeneration comprising, a cell-permeable nucleic acid complex containing a bioactive nucleic acid targeting an NLRP3 gene; and a carrier peptide nucleic acid which are complementarily bound to said bioactive nucleic acid, as an active ingredient.

[Background Art]

Macular degeneration, one of the three major diseases causing blindness, along with glaucoma and diabetic retinopathy, is largely classified into wet (exudative) macular degeneration and dry (non-exudative) macular degeneration. The macula, which is nervous tissue located in the center of the retina, plays an important role in visual acuity. In particular, dry macular degeneration is a disease in which retinal and choroidal atrophy appears due to accumulation of drusen with age. Although not affecting visual acuity, dry macular degeneration is a disease that requires attention because it may progress to wet macular degeneration. Dry macular degeneration mostly occurs due to aging and is regarded as a geriatric disease, but recently, the incidence thereof in young adults has also increased, and the number of macular degeneration patients increased by about 49% in the 5 years from 2011 to 2015 (Health Insurance Review and Assessment Service). The main cause of macular degeneration is aging, followed by genetic predisposition and environmental factors such as UV radiation, smoking, a high-fat and high-calorie westernized diet, and the like.

Typical methods of treating dry (non-exudative) macular degeneration include in taking antioxidant vitamins, which are known to slow the progression of macular degeneration, treating high blood pressure, hyperlipidemia, etc., which are risk factors for macular degeneration, and lifestyle changes such as smoking cessation and UV protection. When dry (non-exudative) macular degeneration progresses to wet (exudative) macular degeneration, active treatment is required in order to preserve visual acuity. Thermal laser photocoagulation is performed when the boundary of the area where degeneration has occurred is clearly identified, and photodynamic therapy, antibody injection, vitrectomy, and the like are also conducted, but no complete therapy has been reported to date, and thorough research thereon is ongoing.

In the case of progressing from dry (non-exudative) macular degeneration to wet (exudative) macular degeneration, thermal laser coagulation is generally performed to prevent long-term loss of visual acuity, but immediately after laser treatment, the retina is damaged and visual acuity is deteriorated, which is undesirable. More recently, photodynamic therapy (PDT), which uses a drug and a laser together to help stabilize the neovascular lesion, has been developed to overcome this problem, but has limitations in that the treatment is difficult to adapt, therapeutic drugs are expensive, and repeated treatment is often required.

Recently, a method of injecting an anti-vascular endothelial growth factor antibody (anti-VEGF antibody) into the eye is mainly used. Representative drugs include aflibercept (Eylea), ranibizumab (Lucentis), and bevacizumab (Avastin). Compared to older forms of treatment, it is possible to maintain visual acuity in a large number of patients, and it has been reported that visual acuity may be improved in some cases. However, this method is inconvenient in that injection must be repeatedly performed every 4 to 8 weeks, and has the disadvantage of being expensive.

Meanwhile, NLRP3 is a protein involved in the inflammatory response and plays a major role in the expression of IL-1β and IL-18. NLRP3 (NOD-like receptor family, pyrin domain-containing 3) constitutes an inflammasome, which is a caspase-1 activation composite protein complex. The inflammasome is known to be composed of NLRP3 (NOD-like receptor family, pyrin domain-containing 3), which is a sensor protein, ASC (adaptor protein apoptosis-associated spec-like protein containing a caspase-recruitment domain), which is an adapter protein, and pro-caspase-1, which is an effector protein. The inflammasome, activated when assembled, activates caspase-1 and secretes IL-1_ or IL-18 to induce an inflammatory response. The association between NLRP3 inflammasome activation and retinal disease (dry and wet age-related macular degeneration) has been previously reported in several documents (Lucia Celkova et al., NLRP3 Inflammasome and Pathobiology in AMD, J. Clin. Med. 2015 Jan; 4(1): 172-192). In addition, dry (non-exudative) macular degeneration is characterized by the presence of excessive amounts of drusen, and Sarah Doyle and Matthew Campbell have reported that drusen accumulated in the macula may lead to the production of IL-18 and IL-1β through an inflammatory response and also that IL-18 or the like causes progression from dry macular degeneration to wet macular degeneration. Moreover, NLRP3 is associated with accumulation of AluRNA and loss of Dicer-1, and activates caspase-1 together with NLRP3 inflammasome to thus induce retinal pigment epithelial degeneration, which has been reported to appear very similar to geographic atrophy *in vivo* (Valeria Tarallo et al., Cell 2012 May 11;149(4):847-59).

Unlike traditional drugs, nucleic acid drugs inhibit the expression of target-specific messenger RNA (mRNA), making it possible to address areas of research in which treatment with conventional drugs targeting proteins is impossible (Kole R. et al. Nature Rev. Drug Discov. 2012; 11; 125-140., Wilson C. et al. Curr. Opin. Chem. Bio. 2006; 10: 607-614.). Various clinical trials using nucleic acids are underway due to the performance and advantages thereof when used as drugs, and despite increasing application of therapeutics based on nucleic acids, the use of carriers for intracellular introduction has been extremely limited. For example, clinical trials on intracellular or intratissue delivery strategies (methods) of drugs based on oligonucleic acids using nanoparticles, cationic liposomes, and polymeric nanoparticles are underway, but in most cases, direct introduction of nucleic acids via administration routes such as intramuscular injection, ocular administration, subcutaneous injection, and the like, which are parenteral methods not including a delivery system, is mainly used.

Moreover, oligonucleic acids alone have very low cell membrane permeation capability. In particular, since DNA or RNA is negatively charged, it cannot pass through the hydrophobic phospholipid bilayer of the cell, so intracellular delivery through simple diffusion is difficult. The use of viral carriers such as retrovirus or AAV (adeno-associated virus) enables the introduction of oligonucleic acids into cells, but there are risks such as unintended immune activity and the possibility of recombination of oncogenes (Couto L.B. et al. Curr. Opin. Pharmacol. 2010, 5; 534-542.).

For this reason, the importance of developing a nucleic acid carrier based on a non-viral oligonucleic acid having low cytotoxicity and low immune activity is continually increasing, and as a result, cell introduction techniques using cationic lipids, liposomes, stable nucleic acid lipid particles (SNALPs), polymers, and cell-penetrating peptides are being developed (Zhi D. et al., Bioconjug. Chem. 2013, 24; 487-519., Buyens K. et al., J. Control Release, 2012, 158; 362-70., ROSSI, J. J. et al., Gene Ther. 2006, 13: 583-584., Yousefi A. et al., J. Control Release, 2013, 170; 209-18., Trabulo S. et al., Curr. Pharm. Des. 2013, 19; 2895-923.).

Such nucleic acid delivery techniques impart a functional moiety through direct bonding, and include a step for forming a complex, but have problems related to endosomal escape efficiency of the liposome structure and biotoxicity. It is necessary to improve the oligonucleic acid introduction function and solve problems related to production procedures and side effects.

In this regard, the present inventors found that the cell permeation capability of a nucleic acid complex in which a bioactive nucleic acid and a carrier peptide nucleic acid modified to have an overall positive charge are complementarily bound to each other may be greatly improved, and that the expression of a target gene may be very efficiently regulated using the same, and have been granted a patent for a new nucleic acid construct having low cytotoxicity and improved cell permeation and gene expression control capability of a bioactive nucleic acid (Korean Patent No. 10-1963885).

The present inventors have continuously conducted research on improvements in the cell delivery function of the construct and therapeutic drugs, and have found that a nucleic acid complex in which a bioactive nucleic acid and a carrier peptide nucleic acid modified to have an overall positive charge are complementarily bound to each other is able to cross the skin and cells very efficiently, indicating that this nucleic acid complex has excellent cell permeation capability.

Accordingly, the present inventors have made great efforts to develop a nucleic acid complex capable of effectively treating macular degeneration through a simple method such as administration of eye drops without direct injection by improving ocular cell permeation capability, and ascertained that a cell-permeable nucleic acid complex in which a bioactive nucleic acid targeting the NLRP3 gene and a carrier peptide nucleic acid are complementarily bound to each other is very effective for the prevention or treatment of macular degeneration, thus culminating in the present invention.

[Disclosure]

It is an object of the present invention to provide a pharmaceutical composition for preventing, ameliorating, or treating macular degeneration, which contains, as an active ingredient, a nucleic acid complex having high cell permeation capability and excellent preventive or therapeutic effects on macular degeneration.

In order to accomplish the above object, the present invention provides a pharmaceutical composition for preventing, ameliorating, or treating macular degeneration comprising, a cell-permeable nucleic acid complex containing a bioactive nucleic acid targeting an NLRP3 gene; and a carrier peptide nucleic acid which are complementarily bound to said bioactive nucleic acid, as an active ingredient.

In addition, the present invention provides a method of preventing or treating macular degeneration including administering the cell-permeable nucleic acid complex.

In addition, the present invention provides the use of the cell-permeable nucleic acid complex for the prevention or treatment of macular degeneration.

In addition, the present invention provides the use of the cell-permeable nucleic acid complex for the manufacture of a medicament for the prevention or treatment of macular degeneration.

[Description of Drawings]

FIG. 1 shows the results of Western blot assay confirming NLRP3 and downstream gene expression changes over time (24 hours, 48 hours, 72 hours, 96 hours, and 120 hours) after administration of nucleic acid complexes, the complexes (1, 2, 3, 4, and 5) for respective lanes being illustrated in Table 4;

FIG. 2a shows the results of analysis through immunocytochemical staining confirming changes in expression of NLRP3 over time (day 1, day 3, and day 5) after treatment with the nucleic acid complex of Table 5 in a dry human-derived retinal pigment epithelial cell line;

FIG. 2b shows the results of analysis through immunocytochemical staining confirming changes in expression of downstream genes (caspase-1) over time (day 1, day 3, and day 5) after treatment with the nucleic acid complex of Table 5 in a dry human-derived retinal pigment epithelial cell line;

FIG. 3 shows optical microscope images of retinal tissue observed 2 weeks after administration of eye drops containing the nucleic acid complex of Table 6 to macular degeneration-induced mice, in which, in the group treated with the nucleic acid complex, damage to the retinal pigment epithelial cells was inhibited and collapse of the outer nuclear layer and the inner nuclear layer was alleviated;

FIG. 4 shows optical microscope images of retinal tissue observed 4 weeks after administration of eye drops containing the nucleic acid complex of Table 6 to macular degeneration-induced mice, and graphs digitizing accumulation of drusen and choroidal atrophy confirmed through fundus imaging using ImageJ;

FIG. 5a shows optical microscope images of retinal tissue observed 2 weeks after administration of eye drops containing the nucleic acid complex of Table 7 to macular degeneration-induced mice; and

FIG. 5b shows images of retinal sections observed 2 weeks after administration of eye drops containing the nucleic acid complex of Table 7 to macular degeneration-induced mice.

[Mode for Invention]

Unless otherwise defined, all technical and scientific terms used herein have the same meanings as those typically understood by those skilled in the art to which the present invention belongs. In general, the nomenclature used herein and the test methods described below are well known in the art and are typical.

Macular degeneration remains the number one cause of blindness and prevents the elderly from living a healthy full life. Dry macular degeneration is classified into early, middle, and late macular degeneration depending on the presence or absence of drusen, the size and number of drusen, the presence or absence of geographic atrophy, and the extent of invasion thereof. It is known that about 10% of patients with dry macular degeneration progress to wet macular degeneration, which may eventually lead to blindness, so the development of a therapeutic agent for dry macular degeneration is very important. Currently, in cases of dry macular degeneration, it is meaningful to maintain the status quo and inhibit the progression of the disease to wet macular degeneration through use of antioxidant vitamins, treatment for high blood pressure, hyperlipidemia, etc., which are risk factors for macular degeneration, and lifestyle changes such as smoking cessation and UV protection, rather than treatment of the disease itself. There is no effective therapeutic agent.

In the present invention, it has been confirmed that a cell-permeable nucleic acid complex in which a bioactive nucleic acid targeting the NLRP3 gene and a carrier peptide nucleic acid are complementarily bound to each other is useful for the prevention and treatment of macular degeneration.

In an embodiment of the present invention, based on Korean Patent No. 10-1963885, granted to the present inventors, when human retinal pigment epithelial cells are treated with a cell-permeable nucleic acid complex in which a bioactive nucleic acid targeting the NLRP3 gene and a carrier peptide nucleic acid are complementarily bound to each other, it has been confirmed that the expression of NLRP3 and downstream genes was effectively inhibited. In another embodiment of the present invention, in mice, the eyes of which were administered with the nucleic acid complex, it has been confirmed that damage to retinal pigment epithelial cells was inhibited and also that collapse of the outer nuclear layer and the inner nuclear layer was alleviated.

Accordingly, an aspect of the present invention pertains to a pharmaceutical composition for preventing, ameliorating, or treating macular degeneration comprising, a cell-permeable nucleic acid complex containing a bioactive nucleic acid targeting an NLRP3 gene; and a carrier peptide nucleic acid which are complementarily bound to said bioactive nucleic acid, as an active ingredient.

In the present invention, the nucleic acid complex in which the bioactive nucleic acid and the carrier peptide are complementarily bound to each other may have the structure of Structural Formula (1) below.

Structural Formula (1)

[A ≡ C(+)]

In Structural Formula (1),

A represents a bioactive nucleic acid having a sequence capable of binding to a target gene,

C represents a carrier peptide nucleic acid capable of binding to the bioactive nucleic acid,

'≡' represents complementary binding between the bioactive nucleic acid and the carrier peptide nucleic acid,

the bioactive nucleic acid represented by A has an overall negative charge or no charge,

the carrier peptide nucleic acid represented by C(+) has an overall positive charge, and

the carrier peptide nucleic acid includes at least one modified peptide nucleic acid monomer such that the carrier peptide nucleic acid has an overall positive charge.

The bioactive nucleic acid and the carrier peptide nucleic acid in the nucleic acid complex according to the present invention are bound through antiparallel binding or parallel binding. In the present invention, the complementary binding form of nucleic acid is configured such that the bioactive nucleic acid is released in the presence of a target sequence (a sequence complementary to the bioactive nucleic acid).

In the present invention, the carrier peptide nucleic acid preferably includes at least one gamma- or alpha-backbone-modified peptide nucleic acid monomer such that the carrier peptide nucleic acid has an overall positive charge, and more preferably, the gamma- or alpha-backbone-modified peptide nucleic acid monomer includes monomers having positively charged amino acids, the number of which is higher than the number of monomers having negatively charged amino acids, so the carrier peptide nucleic acid has an overall positive charge.

In the present invention, the "bioactive nucleic acid" binds *in vitro* or *in vivo* to a target gene and a base sequence including the same, and thus activates or inhibits the intrinsic function of the gene (e.g. transcript expression or protein expression), or regulates splicing of pre-mRNA (e.g. exon skipping). The base sequence may be a gene regulatory sequence, a gene-coding sequence, or a splicing regulatory sequence. Preferably, the bioactive nucleic acid is a nucleic acid having a complementary sequence capable of binding to a target gene of interest to reduce expression thereof, particularly a complementary sequence capable of binding to mRNA of the target gene of interest, and is a nucleic acid involved in gene expression regulation, such as suppressing gene expression, and the like. It may be a nucleic acid having a sequence complementary to a target gene, expression of which is to be reduced.

Therefore, the "bioactive nucleic acid" in the present invention is preferably an antisense peptide nucleic acid of the NLRP3 (NOD-like receptor family, pyrin domain-containing 3) gene, which is a target gene related to macular degeneration, and more preferably includes the sequence of SEQ ID NO: 4 of Table 1 below, but is not limited thereto.

[Table 1]

| Classification | Sequence number | Base sequence | Monomer modification |
|---|---|---|---|
| Bioactive nucleic acid | SEQ ID NO: 4 | 5'-TCG$^{(-)}$AT$^{(+)}$CAT$^{(-)}$ TA$^{(+)}$GCG$^{(-)}$TG-O-K-3' | -+-+- |

The bioactive nucleic acid may be selected from the group consisting of DNA, RNA, modified nucleic acids, such as PNA (peptide nucleic acid), PMO (phosphorodiamidate morpholino oligonucleotide), LNA (locked nucleic acid), GNA (glycol nucleic acid), and TNA (threose nucleic acid), antisense oligonucleotides, aptamers, siRNA (small interfering RNA), shRNA (short hairpin RNA), ribozymes, and DNAzymes, and the bioactive nucleic acid is preferably selected from the group consisting of DNA, RNA, and modified nucleic acids, such as PNA (peptide nucleic acid), PMO (phosphorodiamidate morpholino oligonucleotide), LNA (locked nucleic acid), GNA (glycol nucleic acid), and TNA (threose nucleic acid).

In the present invention, the "carrier peptide nucleic acid" is a nucleic acid in which some or all bases are complementarily bound to the bioactive nucleic acid to thus impart functionality thereto, and the carrier peptide nucleic acid as used herein may include a peptide nucleic acid (PNA) and modified nucleic acids similar thereto, with a peptide nucleic acid being preferred, but the present invention is not limited thereto.

In particular, in the present invention, the carrier peptide nucleic acid is preferably represented by any sequence selected from the group consisting of SEQ ID NOS: 5 to 11 shown in Table 2 below, but is not limited thereto.

[Table 2]

| Classification | Sequence number | Peptide facilitating endosomal escape | Base sequence | Monomer modification |
|---|---|---|---|---|
| Carrier peptide nucleic acid | 5 | Histidine(10) | 5'-Histidine(10)-O-TGGA(+)GATG(+)CGTT(+)AGG-O-K-3' | +++ |
| | 6 | - | 5'-TGGA(+)GATG(+)CGTT(+)AGG-O-K-3' | +++ |
| | 7 | Histidine(10) | 5'-K-O-CA(+)CG(+)C-O-Histidine(10)-3' | ++ |
| | 8 | Histidine(10) | 5'-Histidine(10)-O-CG(+)CA(+)C-O-K-3' | ++ |
| | 9 | Histidine(10) | 5'-K-O-CA(+)CGCTA(+)ATGAT(+)CGA-O-Histidine(10)-3' | +++ |
| | 10 | Histidine(10) | 5'-Histidine(10)-O-AGC(+)TAGTA(+)ATCGC(+)AC-O-K-3' | +++ |
| | 11 | - | 5'-AGC(+)TAGTA(+)ATCGC(+)AC-O-K-3' | +++ |

In the present invention, the bioactive nucleic acid and the carrier peptide nucleic acid may further include at least one functional group selected from the group consisting of phosphodiester, 2'0-methyl, 2'methoxy-ethyl, phosphoramidate, methylphosphonate, and phosphorothioate.

In the present invention, the "cell-permeable nucleic acid complex" enables a bioactive material to penetrate into the body, and ultimately into cells, through extracellular treatment, and specifically has the ability to deliver a bioactive nucleic acid targeting the NLRP3 gene into cells.

Depending on the net charge in the cell-permeable nucleic acid complex according to the present invention and/or the number of bioactive nucleic acids and/or carrier peptide nucleic acids in the nucleic acid complex, the nucleic acid complex may remain in the eye, or may pass through cells and be delivered into the body.

Accordingly, in the present invention, the nucleic acid complex may be characterized as having ocular retention.

In particular, the "cell-permeable nucleic acid complex" according to the present invention is able to be delivered into a desired cell or into an ocular cell in the present invention through, in addition to direct administration into cells, dropping into the eye, intraretinal administration, intraperitoneal administration, intravenous administration, intramuscular administration, subcutaneous administration, intradermal administration, oral administration, intranasal administration, intrapulmonary administration, intrarectal administration, or transdermal administration, and may be used in any form containing the nucleic acid complex.

In the present invention, the cell-permeable nucleic acid complex preferably includes a bioactive nucleic acid represented by SEQ ID NO: 4 and a carrier peptide nucleic acid represented by any one sequence selected from the group consisting of SEQ ID NOS: 5 to 11, but is not limited thereto.

In addition, the binding affinity (melting temperature, Tm) between the bioactive nucleic acid targeting the NLRP3 gene and the carrier peptide nucleic acid is lower than the binding affinity between the bioactive nucleic acid and the NLRP3 gene, which is the target of the bioactive nucleic acid.

The binding affinity between the bioactive nucleic acid and the carrier peptide nucleic acid is based on parallel binding or partial specific binding depending on the 5' orientation and the 3' orientation of each nucleic acid, whereby the binding affinity (melting temperature, Tm) between the bioactive nucleic acid and the carrier peptide nucleic acid may be lower than the binding affinity of the bioactive nucleic acid and the target gene of the bioactive nucleic acid.

In the present invention, the bioactive nucleic acid or the carrier peptide nucleic acid may include a material facilitating endosomal escape that is additionally bound to the 5'-end or the 3'-end of each nucleic acid. Specifically, the nucleic acid complex further includes a material facilitating endosomal escape of the bioactive nucleic acid and the carrier peptide nucleic acid, and thus has the structure of Structural Formula (2) below.

Structural Formula (2)

[mA ≡ mC(+)]

In Structural Formula (2),
'm' represents a material facilitating endosomal escape of the bioactive nucleic acid and the carrier peptide nucleic acid.

In the present invention, the "material facilitating endosomal escape" facilitates escape of the bioactive nucleic acid from the endosome by increasing osmotic pressure inside the endosome or destabilizing the endosomal membrane. This means that the bioactive nucleic acid moves more efficiently and quickly into the nucleus or cytoplasm to meet and act on a target gene (D.W. Pack, A.S. Hoffman, S. Pun, P.S. Stayton, "Design and development of polymers for gene delivery," Nat. Rev. Drug. Discov., 4, 581-593 (2005)).

In the present invention, the material facilitating endosomal escape is at least one selected from the group consisting of a peptide, lipid nanoparticles, polyplex nanoparticles, polymer nanospheres, inorganic nanoparticles, cationic lipid-based nanoparticles, a cationic polymer, and a pH-sensitive polymer.

In the present invention, as the material facilitating endosomal escape, a peptide (GLFDIIKKIAESF, SEQ ID NO: 12) may be linked to the bioactive nucleic acid via a linker, and histidine (10) may be linked to the carrier peptide nucleic acid via a linker, but the present invention is not limited thereto.

In the present invention, the lipid nanoparticles may be selected from the group consisting of lipids, phospholipids, acetyl palmitate, Poloxamer 18, Tween 85, tristearin glyceride, and Tween 80.

In the present invention, the polyplex nanoparticles may be poly(amidoamine) or polyethyleneimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of $Fe_2O_3$, $Fe_3O_4$, $WO_3$, and $WO_{2.9}$.

In the present invention, the cationic lipid-based nanoparticles may be selected from the group consisting of 1-(aminoethyl)iminobis[N-(oleicylcysteinyl-1-amino-ethyl)propionamide], N-alkylated derivatives of PTA, and 3,5-didodecyloxybenzamidine.

In the present invention, the cationic polymer may be selected from the group consisting of vinylpyrrolidone-N,N-dimethylaminoethyl methacrylate acid copolymer diethyl sulfate, polyisobutylene, and poly(N-vinylcarbazole).

In the present invention, the pH-sensitive polymer may be selected from the group consisting of polyacids, poly(acrylic acid), poly(methacrylic acid), and hydrolyzed polyacrylamide.

In the present invention, each of the bioactive nucleic acid and the carrier peptide nucleic acid includes 2 to 50, preferably 5 to 30, more preferably 10 to 25, and most preferably 15 to 17 nucleic acid monomers.

The bioactive nucleic acid may be composed of a natural nucleobase and/or a modified nucleic acid monomer.

In the present invention, when the monomer used for the bioactive nucleic acid is PNA, it is called a bioactive nucleic acid, and when other monomers are used, they are referred to in the same manner.

In the present invention, the bioactive nucleic acid and the carrier peptide nucleic acid may further include at least one functional group selected from the group consisting of phosphodiester, 2'0-methyl, 2'methoxy-ethyl, phosphoramidate, methylphosphonate, and phosphorothioate.

In the present invention, the carrier peptide nucleic acid may be composed of a sequence in which part or all of the base sequence is complementary to the bioactive nucleic acid. In particular, the carrier peptide nucleic acid may include at least one universal base, and the carrier peptide nucleic acid may be composed exclusively of universal bases.

In the present invention, each of the bioactive nucleic acid and the carrier peptide nucleic acid in the cell-permeable nucleic acid complex may have an overall positive charge (cationic), negative charge (anionic), or no charge (neutral), with regard to the electrical properties thereof.

The term "overall" used when representing electrical properties means the general electrical properties of the entire bioactive nucleic acid or carrier peptide nucleic acid from an outside perspective, rather than the electrical properties of individual bases. For example, even though some monomers in the bioactive nucleic acid are positively charged, when the number of negatively charged monomers is greater, the bioactive nucleic acid is said to be negatively charged when considering the overall electrical properties, and even though some bases and/or backbones in the carrier peptide nucleic acid are negatively charged, when the number of positively charged bases and/or backbones is greater, the carrier peptide nucleic acid is said to be positively charged when considering the overall electrical properties.

From this point of view, the nucleic acid complex of the present invention preferably has an overall positive charge. In the nucleic acid complex, it is preferred that the bioactive nucleic acid be negatively charged or neutral when viewing the overall electrical properties thereof and that the carrier peptide nucleic acid be positively charged when viewing the overall electrical properties thereof, but the present invention is not limited thereto.

In the present invention, the electrical properties of the bioactive nucleic acid and the carrier peptide nucleic acid may be imparted using a modified peptide nucleic acid monomer, and the modified peptide nucleic acid monomer may include, as a positively charged carrier peptide nucleic acid, at least one positively charged amino acid selected from the group consisting of lysine (Lys, K), arginine (Arg, R), histidine (His, H), diamino butyric acid (DAB), ornithine (Orn), and amino acid analogues, and may include, as a negatively charged carrier peptide nucleic acid, a negatively charged amino acid such as glutamic acid (Glu, E) or a negatively charged amino acid analogue.

In the present invention, the carrier peptide nucleic acid may include at least one gamma- or alpha-backbone-modified peptide nucleic acid monomer such that it has an overall positive charge.

The gamma- or alpha-backbone-modified peptide nucleic acid monomer may include, in the backbone thereof, at least one positively charged amino acid selected from the group consisting of lysine (Lys, K), arginine (Arg, R), histidine (His, H), diamino butyric acid (DAB), ornithine (Orn), and amino acid analogues in order to attain an electrically positive charge.

In the present invention, modification of the peptide nucleic acid monomer so as to be electrically charged may be performed using a nucleobase-modified peptide nucleic acid monomer, in addition to the backbone modification. Preferably, an amine, triazole, or imidazole moiety is included in the nucleobase in order to attain an electrically positive charge, or carboxylic acid is included in the base in order to attain an electrically negative charge.

In the present invention, the modified peptide nucleic acid monomer of the carrier peptide nucleic acid may further include the negative charge in the backbone or nucleobase, but the modified peptide nucleic acid monomer preferably includes positively charged monomers, the number of which is higher than the number of negatively charged monomers, so the carrier peptide nucleic acid has an overall positive charge.

It is preferred that the nucleic acid complex according to the present invention have an overall positive charge.

In the nucleic acid complex according to the present invention, at least one material selected from the group consisting of a hydrophobic moiety, hydrophilic moiety, target antigen-specific antibody, aptamer, and fluorescent/luminescent label may be bound to the bioactive nucleic acid and/or the carrier peptide nucleic acid, and preferably, at least one material selected from the group consisting of a hydrophobic moiety, hydrophilic moiety, target antigen-specific antibody, aptamer, and fluorescent/luminescent label for imaging is bound to the carrier peptide nucleic acid.

In the present invention, at least one material selected from the group consisting of a hydrophobic moiety, hydrophilic moiety, target antigen-specific antibody, aptamer, quencher, fluorescent label, and luminescent label may be bound to the bioactive nucleic acid and/or the carrier peptide nucleic acid through simple covalent bonding or linker-mediated covalent bonding, but the present invention is not limited thereto. Preferably, the material associated with cell permeation capability, solubility, stability, transport, and imaging (e.g. a hydrophobic moiety, etc.) bound to the nucleic acid carrier is present independently of the bioactive nucleic acid that controls the expression of the target gene.

In the present invention, as described above, the complementary binding form of the bioactive nucleic acid and the carrier peptide nucleic acid is largely based on antiparallel binding or parallel binding. The complementary binding form is configured such that the bioactive nucleic acid is released in the presence of a target sequence (a sequence complementary to the bioactive nucleic acid).

The antiparallel binding and the parallel binding are determined depending on the 5' orientation and the 3' orientation in the DNA-DNA or DNA-PNA binding mode. Antiparallel binding is a typical method for DNA-DNA or DNA-PNA binding. Taking the nucleic acid complex according to the present invention as an example, the bioactive nucleic acid in the 5' → 3' orientation and the carrier peptide nucleic acid in the 3' → 5' orientation are bound to each other. Parallel binding is a binding form in which binding affinity is somewhat lower than that of antiparallel binding, and both the bioactive nucleic acid and the carrier peptide nucleic acid are bound to each other in the 5' → 3' orientation or the 3' → 5' orientation.

In the nucleic acid complex according to the present invention, it is preferred that the binding affinity between the bioactive nucleic acid and the carrier peptide nucleic acid be lower than the binding affinity between the bioactive nucleic acid and a target gene of the bioactive nucleic acid, particularly mRNA of the target gene. The binding affinity is determined based on a melting temperature Tm.

An example of a specific method for making the binding affinity (melting temperature, Tm) between the bioactive nucleic acid and the carrier peptide nucleic acid lower than the binding affinity between the bioactive nucleic acid and the target gene of the bioactive nucleic acid, particularly the mRNA of the target gene, may include parallel binding or partial specific binding of the bioactive nucleic acid and the carrier peptide nucleic acid, but the present invention is not limited thereto.

In another example, the carrier peptide nucleic acid may include at least one peptide nucleobase selected from the group consisting of a linker, a universal base, and a peptide nucleobase having a base that is not complementary to the corresponding base of the bioactive nucleic acid, but the present invention is not limited thereto.

In the present invention, the universal base is a base pairing with a natural base such as adenine, guanine, cytosine, thymine, or uracil without selectivity and having binding affinity lower than the complementary binding affinity, and is at least one selected from the group consisting of inosine PNA, indole PNA, nitroindole PNA, and abasic PNA, preferably inosine PNA.

In the present invention, a combination of the binding form and the electrical properties of nucleic acids for controlling the function of the nucleic acid complex is provided, and the combination of the binding form and the electrical properties of nucleic acids enables the particle size and the time of action to be controlled and cell permeation capability, solubility, and specificity to be improved.

In the present invention, it is possible to control the time point at which the bioactive nucleic acid is bound to the target sequence in the presence of the target gene by adjusting the binding affinity between the bioactive nucleic acid and the carrier peptide nucleic acid (the time point of displacement of the strand of the bioactive nucleic acid to the target sequence, and the time point of target-specific release and binding of the bioactive nucleic acid).

In the nucleic acid complex according to the present invention, the time point of displacement of the strand of the bioactive nucleic acid to the target gene and the time point of target-specific release and binding of the bioactive nucleic acid may be controlled by varying the presence, number, and position of non-specific bases, universal bases, and linkers of the carrier peptide nucleic acid for non-specific binding of the complex. Control may be performed through a combination of the above conditions and the complementary binding form of the peptide complex, such as parallel binding or antiparallel binding.

In the present invention, the particles of the nucleic acid complex may have a size of 5 nm to 300 nm, preferably 10 nm to 200 nm, and most preferably 15 nm to 100 nm.

In the present invention, the particle size of the nucleic acid complex may be controlled by adjusting charge balance between the bioactive nucleic acid and the carrier peptide nucleic acid. Specifically, when the positive charge of the carrier peptide nucleic acid increases, the particle size decreases, but when the positive charge of the carrier peptide nucleic acid exceeds a certain level, the particle size increases. In addition, the particle size is determined by appropriate charge balance between the bioactive nucleic acid and the carrier peptide nucleic acid based on the charge of the bioactive nucleic acid forming the complex, as another important factor determining the particle size.

The number of positive charges in the carrier peptide nucleic acid according to the present invention is 1 to 7 (meaning that 1 to 7 positively charged monomers are included), preferably 2 to 5, and most preferably 2 to 3, and the number of negative charges in the bioactive nucleic acid is 0 to 5, and preferably 0 to 3, based on the net charge of charge balance therebetween.

In the present invention, the nucleic acid complex may be constructed by hybridizing a bioactive nucleic acid and a carrier peptide nucleic acid under appropriate conditions.

"Hybridization" in the present invention means that complementary single-stranded nucleic acids form a double-stranded nucleic acid. Hybridization may occur when complementarity between two nucleic acid strands is perfectly matched, or even when some mismatched bases are present. The extent of complementarity required for hybridization may vary depending on the hybridization conditions, in particular the binding temperature.

The term "target gene" when used in the present invention means a nucleic acid sequence (base sequence) to be activated, inhibited, or labeled, is not different from the term "target nucleic acid", and is used interchangeably therewith herein.

In the present invention, when a target nucleic acid (base sequence) including a target gene comes into contact with (binds to) the complex *in vitro* or *in vivo,* the bioactive nucleic acid is separated from the carrier peptide nucleic acid and thus exhibits biological activity.

In the present invention, the diseases that may be prevented or treated using the nucleic acid complex may be determined depending on the target gene of the bioactive nucleic acid in the nucleic acid complex. In the present invention, the target gene of the bioactive nucleic acid is NLRP3.

Therefore, in the present invention, the disease that may be prevented and treated using the nucleic acid complex is preferably macular degeneration, and the macular degeneration includes age-related macular degeneration, dry macular degeneration, and wet macular degeneration. Most preferably, the nucleic acid complex of the present invention is capable of preventing, ameliorating, or treating dry macular degeneration.

In an embodiment of the present invention, the preventive and therapeutic effects on dry macular degeneration were confirmed, but an inflammasome, which is a caspase-1 activation composite protein complex related to the NLRP3 protein, is known to play a major role in progression from dry macular degeneration to wet macular degeneration, so the composition for treatment according to the present invention may also be used to prevent, ameliorate, or treat wet macular degeneration, in particular, to prevent progression from dry macular degeneration to wet macular degeneration (Alexander G. Marneros, 9445-958, 2013; Lucia Celkova et. al., Journal of Clinical Medicine 4(1), 172-192, 2015; Yerramothu P. et al., Eye 32, 491-505, 2018).

In the present invention, the term "composition for treatment" may be used interchangeably with "pharmaceutical composition", and the composition includes, as an active ingredient, the nucleic acid complex of the present invention including the bioactive nucleic acid and the carrier peptide nucleic acid binding to the nucleic acid, and may take a form in which a therapeutic drug for treating a target disease is additionally bound to the nucleic acid complex.

When the pharmaceutical composition is formulated and administered, it may be administered in the form of a formulation produced through a typical method.

The administered formulation may take the form of, for example, an eye drop, eye ointment, powder, granule, tablet, capsule, injection, ointment, and the like, and is preferably in the form of eye drops or skin application, but is not limited thereto. Such formulations may be prepared according to methods common in the art. As an ophthalmic topical dosage form, a drop, spray, or gel form may be provided, and as another method, administration to the eye using liposomes is possible. Injection into the tear film through a pump-catheter system may also be conducted. As an additional embodiment, the composition may be incorporated into, carried by, or attached to contact lenses on the eye. In a further embodiment, a form contained in a sponge or cotton swab capable of being applied to the ocular surface may be used, and a liquid spray capable of being applied to the ocular surface may also be used.

Preferably, the composition according to the present invention is formulated in the form of eye drops, aqueous solutions, gels, ointments, creams, lotions, pastes, smears, or patches.

Most preferably, it is formulated in the form of an aqueous solution, and the aqueous solution may be in the form of distilled water and a buffer solution.

These formulations may contain, in addition to the active ingredient, additives such as carriers, excipients, adjuvants, or diluents suitable for pharmaceutically acceptable formulations, for example formulations that may be applied to the eye or skin.

As used herein, "pharmaceutically acceptable" means that a composition is physiologically acceptable and does not usually cause allergic reactions such as gastrointestinal disorders and dizziness or reactions similar thereto when administered to humans. Examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. The pharmaceutical composition may further include a filler, an anti-aggregation agent, a lubricant, a wetting agent, a flavoring agent, an emulsifier, and a preservative. The pharmaceutical composition of the present invention may also be formulated using methods known in the art so as to provide rapid, sustained, or delayed release of the active ingredient after administration to a mammal. The formulation may be in the form of a sterile injectable solution or the like.

The term "carrier" refers to a compound that facilitates the addition of a nucleic acid complex into a cell or tissue. For example, dimethylsulfoxide (DMSO) is a commonly used carrier that facilitates the introduction of many organic compounds into cells or tissues of living organisms.

The term "diluent" refers to a compound that stabilizes the biologically active form of a compound of interest and is also diluted in water in which the compound is dissolved. A salt dissolved in a buffer solution is used as a diluent in the art. A commonly used buffer solution is phosphate-buffered saline, because it mimics the salinity of human fluids. Since buffer salts may control the pH of a solution at low concentrations, buffer diluents seldom modify the biological activity of a compound.

The material containing the nucleic acid complex according to the present invention may be administered to a patient as a pharmaceutical composition alone or in combination with other active ingredients, as in combination therapy, or with suitable carriers or excipients.

A pharmaceutical composition suitable for use in the present invention includes a composition in which the active ingredients are contained in amounts effective to achieve the intended purposes thereof. More specifically, a therapeutically effective amount is an amount of a compound that is effective in prolonging the lifetime of the subject to be treated, or in preventing, alleviating, or reducing the symptoms of a disease. The determination of a therapeutically effective amount is within the capability of those skilled in the art, particularly in light of the detailed disclosure provided herein.

As used herein, the term "prevention" refers to any action that prevents the onset of a disease or inhibits progression thereof through administration (or application) of the composition for treatment including the cell-permeable nucleic acid complex.

As used herein, the term "amelioration" refers to any action of at least reducing a parameter related to the status of a disease to be treated, for example, the extent of symptoms, through administration (or application) of the composition for treatment including the cell-permeable nucleic acid complex.

As used herein, the term "treatment" refers to any action by which symptoms of a disease are ameliorated or eliminated through administration (or application) of the composition for treatment including the cell-permeable nucleic acid complex.

For administration (or application) of the nucleic acid complex of the present invention, any nucleic acid delivery method known in the art may be used. Suitable delivery reagents include, but are not limited to, for example, Mirus Transit TKO lipophilic reagent, Lipofectin, lipofectamine, Cellfectin, polycations (e.g. polylysine), atelocollagen, nanoplexes, and liposomes. The use of atelocollagen as a delivery carrier for nucleic acid molecules is described in Minakuchi et al. Nucleic Acids Res., 32(13):e109 (2004); Hanai et al. Ann. NY Acad. Sci., 1082:9-17 (2006); and Kawata et al. Mol. Cancer Ther., 7(9):2904-12 (2008). Exemplary interfering nucleic acid delivery systems are disclosed in U.S. Patent Nos. 8,283,461, 8,313,772, and 8,501,930.

In the present invention, the cell-permeable nucleic acid complex may be administered (or applied) using a carrier such as a liposome. The liposome may help to target the complex to a specific tissue, such as lymphoid tissue, or to selectively target an infected cell, and may also help to increase the half-life of a composition including the complex. Examples of liposomes include emulsions, foams, micelles, insoluble monolayers, liquid crystals, phospholipid dispersions, lamellar layers, and the like. In such formulations, the complex to be delivered is incorporated as part of liposomes, either alone or together with molecules that bind to predominant receptors in lymphoid cells, such as monoclonal antibodies that bind to CD45 antigen, in specific cells, or in combination with other therapeutic compositions. Thus, liposomes filled or decorated with a given complex of the present invention to deliver the nucleic acid complex composition may be directed to the site of lymphocytes.

Liposomes for use in accordance with the present invention are generally formed from standard vesicle-forming lipids including neutral and negatively charged phospholipids and sterols such as cholesterol. In general, lipids are selected in consideration of, for example, the stability of the liposome in the bloodstream, acid lability, and the size of the liposome. Various methods may be used for preparing liposomes. For example, methods described in [Szoka et al., Ann. Rev. Biophys. Bioeng., 9:467, 1980 and U.S. Patent Nos. 4,235,871, 4,501,728, 4,837,028, and 5,019,369] may be used.

Another aspect of the present invention pertains to a method of preventing or treating macular degeneration including administering to a subject a cell-permeable nucleic acid complex in which a bioactive nucleic acid targeting the NLRP3 gene and a carrier peptide nucleic acid are complementarily bound to each other.

The composition including the nucleic acid complex according to the present invention may be applied to the eye in a pharmaceutically effective amount in order to treat macular degeneration or to suppress (or alleviate) symptoms of macular degeneration. The pharmaceutically effective amount may vary depending on various factors, such as the type of macular degeneration, the age and body weight of the patient, characteristics and extent of symptoms, therapy currently being administered, the number of types of therapy, the form and route of application, etc., and may be easily determined by experts in the field. The composition of the present invention may be applied together or sequentially with the aforementioned pharmacological or physiological components, and may also be applied in combination with additional conventional therapeutic agents, or may be applied sequentially or simultaneously with conventional therapeutic agents. These applications may be carried out one or multiple times.

In the present invention, the "subject" is a mammal, preferably a human, suffering from or at risk of developing a condition or disease that may be alleviated, suppressed, or treated by administering (or applying) the cell-permeable nucleic acid complex according to the present invention.

In addition, the dosage (application amount) of the compound of the present invention for a human body may vary depending on the patient's age, body weight, and gender, administration (application) form, state of health, and severity of disease, and based on an adult patient weighing 70 kg, the compound may typically be administered in a dose of 0.001 to 1,000 mg/day, and preferably 0.01 to 500 mg/day, and may be administered (applied) once a day or several times a day at regular intervals according to the judgment of a doctor or pharmacist.

The toxicity and therapeutic efficacy of the composition including the cell-permeable nucleic acid complex as described herein may be estimated by standard pharmaceutical procedures in cell culture or experimental animals in order to determine, for example, an LD50 (a dose lethal to 50% of the population), an ED50 (a dose having a therapeutic effect on 50% of the population), or an IC50 (a dose having a treatment inhibitory effect on 50% of the population). The dose ratio between the toxicity and the therapeutic effect is a therapeutic index, which may be represented as the ratio between LD50 and ED50 (or IC50). Compounds exhibiting high therapeutic indices are preferred. Data obtained from cell culture assays may be used to estimate a range of doses for use in humans. The dosages of these compounds or the application amounts thereof preferably fall within a range of circulating concentrations that include ED50 (or IC50) with little or no toxicity.

As used herein, the term "administering" or "administration" refers to an action of introducing the pharmaceutical composition of the present invention to a subject through any suitable method, and the route of administration may be varied, and may be either oral or parenteral, so long as it is able to reach the target tissue.

The pharmaceutical composition of the present invention may be administered through any general route, so long as it is able to reach the target tissue. Depending on the purpose, the pharmaceutical composition of the present invention may be administered through dropping into the eye, intraretinal administration, intraperitoneal administration, intravenous administration, intramuscular administration, subcutaneous administration, intradermal administration, oral administration, intranasal administration, intrapulmonary administration, or intrarectal administration, but the present invention is not particularly limited thereto. Furthermore, the composition may be administered using any device capable of transporting the active material to a target cell.

The pharmaceutical composition of the present invention may be administered in a pharmaceutically effective amount. In the present invention, the term "pharmaceutically effective amount" is an amount sufficient to treat or prevent a disease at a reasonable benefit/risk ratio applicable to medical treatment or prevention, and the effective dose level may be determined depending on factors including the severity of disease, drug activity, patient's age, body weight, health, gender, and drug sensitivity, administration time of the composition of the present invention, administration route, excretion rate, duration of treatment, and drugs used in combination or simultaneously with the composition of the present invention, and other factors well-known in the medical field.

The pharmaceutical composition according to the present invention may be administered as an individual therapeutic agent or in combination with other therapeutic agents, may be administered sequentially or simultaneously with conventional therapeutic agents, and may be administered one or multiple times. Taking into consideration all of the above factors, it is important to administer the composition in the minimum amount capable of obtaining the maximum effect without side effects.

The dosage of the pharmaceutical composition of the present invention may be determined by those skilled in the art in consideration of the purpose of use, the severity of disease, the patient's age, body weight, gender, and disease history, or the type of material used as an active ingredient. For example, the pharmaceutical composition of the present invention may be administered to mammals including humans in an amount of 10 to 100 mg/kg, and preferably 10 to 30 mg/kg, per day. The dose of the composition of the present invention may be divided into multiple doses to realize an administration frequency of 1 to 3 times a day or several times a day, but is not particularly limited thereto.

Still another aspect of the present invention pertains to the use of the cell-permeable nucleic acid complex in which the bioactive nucleic acid targeting the NLRP3 gene and the carrier peptide nucleic acid are complementarily bound to each other for the prevention or treatment of macular degeneration.

Preferably, the macular degeneration is dry macular degeneration or wet macular degeneration, and most preferably dry macular degeneration.

Yet another aspect of the present invention pertains to the use of the cell-permeable nucleic acid complex in which the bioactive nucleic acid targeting the NLRP3 gene and the carrier peptide nucleic acid are complementarily bound to each other for the manufacture of a medicament for the prevention or treatment of macular degeneration.

Preferably, the macular degeneration is dry macular degeneration or wet macular degeneration, and most preferably dry macular degeneration.

Examples

A better understanding of the present invention may be obtained through the following examples. These examples are merely set forth to illustrate the present invention, and are not to be construed as limiting the scope of the present invention, as will be apparent to those skilled in the art.

Example 1: Bioactive nucleic acid, carrier peptide nucleic acid, and construction of complex using these nucleic acids

In order to evaluate the effect of the nucleic acid complex of the present invention on dry macular degeneration, the NLRP3 gene was used as a target gene. In order to confirm the therapeutic effect on dry macular degeneration, an antisense peptide nucleic acid (antisense PNA) was used as a bioactive peptide nucleic acid targeting the NLRP3 gene.

The bioactive peptide nucleic acid (antisense PNA) used as a control in the present invention consists of the sequence represented by SEQ ID NOS: 1 and 2, and the bioactive peptide nucleic acid (antisense PNA) used to confirm the therapeutic effect on dry macular degeneration consists of the sequence represented by SEQ ID NOS: 3 and 4.

Among the PNA-based bioactive nucleic acids used in this Example, SEQ ID NOS: 1 and 3 were synthesized in a form in which a peptide GLFDIIKKIAESF (SEQ ID NO: 12) facilitating endosomal escape was bound to the 5'-end thereof, and SEQ ID NOS: 2 and 4 were synthesized in a form in which the peptide facilitating endosomal escape was not bound thereto. All of the carrier peptide nucleic acids used in this Example, except for those of SEQ ID NOS: 6 and 11, are configured such that the peptide facilitating endosomal escape was bound to the 5'- or 3'-end thereof, and consist of respective sequences described. The base sequences, monomer modifications, and structures are shown in Table 3 below.

All peptide nucleic acids used in the present invention were synthesized through HPLC purification by Panagene (Korea) (Table 3).

[Table 3]

| Sequences of bioactive nucleic acid and carrier peptide nucleic acid for evaluating therapeutic effect on dry macular degeneration | | | | |
|---|---|---|---|---|
| Classification | SEQ ID NO: | Peptide facilitating endosomal escape | Base sequence | Monomer modification |
| Bioactive nucleic acid | 1 | GLFDIIKKIAESF | 5′-GLFDIIKKIAESF-O-AC$^{(-)}$CT$^{(+)}$CTA$^{(-)}$CGC$^{(+)}$AAT$^{(-)}$CC-O-K-3′ | -+-+- |
| | 2 | - | 5′-AC$^{(-)}$CT$^{(+)}$CTA$^{(-)}$CGC$^{(+)}$AAT$^{(-)}$CC-O-K-3′ | -+-+- |
| | 3 | GLFDIIKKIAESF | 5′-GLFDIIKKIAESF-O-TCG$^{(-)}$AT$^{(+)}$CAT$^{(-)}$TA$^{(+)}$GCG$^{(-)}$TG-O-K-3′ | -+-+- |
| | 4 | - | 5′-TCG$^{(-)}$AT$^{(+)}$CAT$^{(-)}$TA$^{(+)}$GCG$^{(-)}$TG-O-K-3′ | -+-+- |
| Carrier peptide nucleic acid | 5 | Histidine(10) | 5′-Histidine(10)-O-TGGA$^{(+)}$GATG$^{(+)}$CGTT$^{(+)}$AGG-O-K-3′ | +++ |
| | 6 | - | 5′-TGGA$^{(+)}$GATG$^{(+)}$CGTT$^{(+)}$AGG-O-K-3′ | +++ |
| | 7 | Histidine(10) | 5′-K-O-CA$^{(+)}$CG$^{(+)}$C-O-Histidine(10)-3′ | ++ |
| | 8 | Histidine(10) | 5′-Histidine(10)-O-CG$^{(+)}$CA$^{(+)}$C-O-K-3′ | ++ |
| | 9 | Histidine(10) | 5′-K-O-CA$^{(+)}$CGCTA$^{(+)}$ATGAT$^{(+)}$CGA-O-Histidine(10)-3′ | +++ |
| | 10 | Histidine(10) | 5′-Histidine(10)-O-AGC$^{(+)}$TAGTA$^{(+)}$ATCGC$^{(+)}$AC-O-K-3′ | +++ |
| | 11 | - | 5′-AGC$^{(+)}$TAGTA$^{(+)}$ATCGC$^{(+)}$AC-O-K-3′ | +++ |

Table 3 shows the sequence information of the bioactive nucleic acid and carrier peptide nucleic acid, as well as those of the bioactive nucleic acid and carrier peptide nucleic acid used as a control, in order to confirm the effect as a therapeutic agent for dry macular degeneration targeting the NLRP3 gene.

Monomer modification for imparting electrical properties was carried out such that a modified peptide backbone was constructed by including, in the backbone of the peptide nucleic acid, lysine (Lys, K, (+)) in order to attain an electrically positive charge and glutamic acid (Glu, E, (-)) in order to attain an electrically negative charge.

The bioactive nucleic acid and the carrier peptide nucleic acid were hybridized under DMSO, and as a result, a complex composed of the bioactive nucleic acid and the carrier peptide nucleic acid was constructed.

Example 2: Analysis of therapeutic effect on dry macular degeneration using nucleic acid complex

The therapeutic effect on dry macular degeneration was analyzed using the nucleic acid complex including the bioactive nucleic acid targeting the NLRP3 gene and the carrier peptide nucleic acid constructed according to Example 1.

Example 2-1: Cell culture

Human retinal pigment epithelium (ARPE-19, ATCC NO. CRL-2302) obtained from ATCC (American Type Culture Collection, USA) was cultured at 37°C and 5% (v/v) $CO_2$ in a DMEM:F-12 culture medium (Dulbecco's Modified Eagle Medium Nutrient Mixture F-12 (Ham), Gibco BRL, Grand Island, NY, USA) supplemented with 10% (v/v) fetal bovine serum, 100 units/ml of penicillin, and 100 $\mu$g/ml of streptomycin. In order to make a cell model resembling dry macular degeneration, the cells were treated with 4 ng/ml of IL (interleukin)-la and 50 ng/ml of LPS (lipopolysaccharide) and cultured for 24 hours.

Example 2-2: Analysis of gene expression through Western blot assay

A human-derived retinal pigment epithelial cell line was seeded at $1 \times 10^5$ cells in a 6-well plate, cultured for 24 hours, treated with the complex including the bioactive peptide nucleic acid and the carrier peptide nucleic acid through experimentation under the conditions of Example 2-1, and cultured for 24, 48, 72, 96, and 120 hours, after which 30 $\mu$L of RIPA buffer was added to each well to obtain a protein lysate. The protein lysate was subjected to protein quantification using a BCA assay kit (Thermo Fisher, USA), and 30 $\mu$g of protein was separated by size through electrophoresis, transferred to a PVDF membrane, treated with primary antibodies such as NLRP3 (Abcam, USA), ASC (Santa Cruz Biotechnology, USA), procaspase-1, caspase-1 (Santa Cruz Biotechnology, USA), proIL-1$\beta$, and IL-1$\beta$ (Abcam, USA) at 1:1000, and allowed to stand at 4°C for one day.

After washing using 1X TBS-T, the cells were treated with secondary antibodies such as Anti-Rabbit (Cell Signaling Technology, USA) and Anti-Mouse (Santa Cruz Biotechnology, USA) at 1:2000 and allowed to stand at room temperature for 1 hour. The cells were treated using a Supersignal™ West Femto Maximum Sensitivity Substrate (Thermo Fisher, USA), and the target gene expression inhibitory efficiency was analyzed in the human retinal pigment epithelial cell line using an Image600 system (Amersham, Germany).

In this Example, changes in expression of NLRP3 and downstream genes in a cell model simulating dry macular degeneration using IL-1$\alpha$ were analyzed, and the nucleic acid complex combinations that were used are given in Table 4 below.

[Table 4]

| Nucleic acid complex used for NLRP3 and downstream gene expression analysis in retinal pigment epithelial cell line (ARPE-19) | |
| --- | --- |
| Nucleic acid complex | Combination of bioactive nucleic acid and carrier peptide nucleic acid |
| 1 | SEQ ID NOS: 1 and 5 |
| 2 | SEQ ID NOS: 3 and 7 |
| 3 | SEQ ID NOS: 3 and 8 |
| 4 | SEQ ID NOS: 3 and 9 |
| 5 | SEQ ID NOS: 3 and 10 |

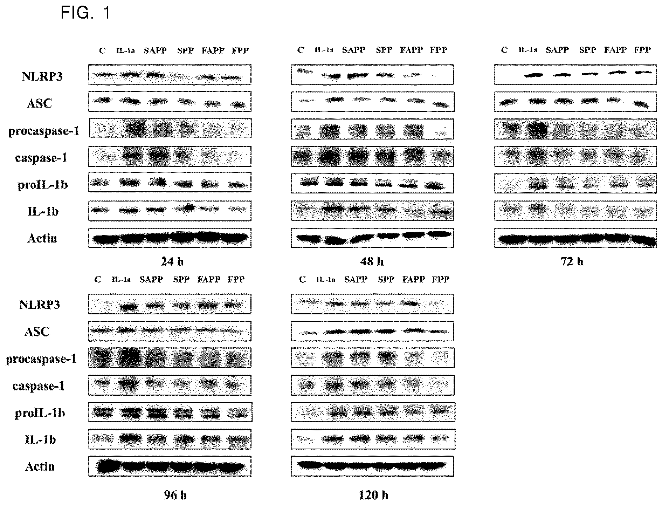

Based on the results thereof, as shown in FIG. 1, the nucleic acid complexes obtained through the combination of SEQ ID NO: 3 and SEQ ID NO: 8 and the combination of SEQ ID NO: 3 and SEQ ID NO: 10 were confirmed to most strongly inhibit, over time, the expression of the target gene and the expression of the downstream genes.

Example 2-3: Immunocytochemical staining analysis

A human-derived retinal pigment epithelial cell line was seeded at $5 \times 10^3$ cells in an 8-well plate, cultured for 24 hours, treated with the complex including the bioactive peptide nucleic acid and the carrier peptide nucleic acid through experimentation under the conditions of Example 2-1, cultured for 24, 48, and 72 hours, fixed in 4% paraformaldehyde, blocked with 0.1% Triton X-100 and normal donkey serum, treated using primary antibodies such as NLRP3 (Abcam, USA) and caspase-1 (Santa Cruz Biotechnology, USA) at 1:200, and allowed to stand at 4°C for one day.

Thereafter, the cells were treated with secondary antibodies such as Donkey Anti-Goat IgG H&L (Alexa Fluor® 647) (Abcam, USA) and Goat Anti-Mouse IgG H&L (Alexa Fluor® 488) (Abcam, USA) at 1:200, allowed to stand at room temperature for 1 hour, and fixed using a mounting agent containing DAPI, and the results thereof were observed using a microscope.

In this Example, changes in expression of NLRP3 and downstream genes in a cell model simulating dry macular degeneration using LPS were analyzed, and the nucleic acid complex combinations that were used are given in Table 5 below.

[Table 5]

| Nucleic acid complex combination for analysis of therapeutic effect on dry macular degeneration through immunocytochemical staining | |
|---|---|
| Classification | Nucleic acid complex |
| 1 | SEQ ID NOS: 1 and 5 |
| 2 | SEQ ID NOS: 3 and 8 |
| 3 | SEQ ID NOS: 3 and 10 |

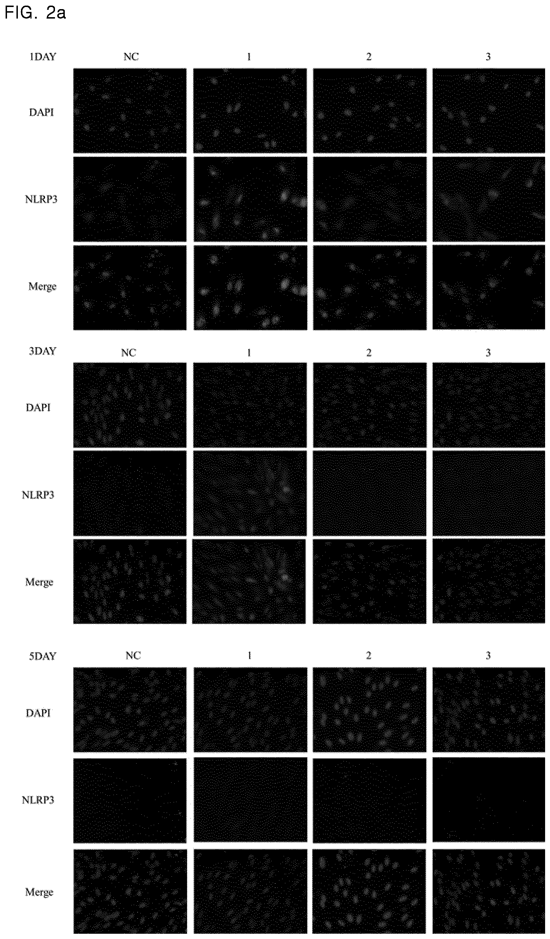

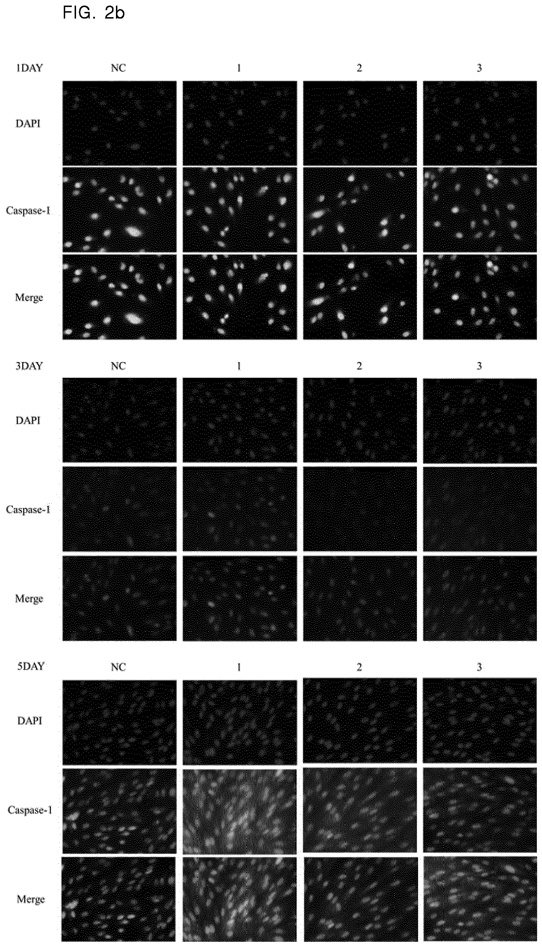

Based on the results thereof, as shown in FIGS. 2a and 2b, the nucleic acid complex obtained through the combination of SEQ ID NO: 3 and SEQ ID NO: 10 was confirmed to most strongly inhibit, over time, the expression of NLRP3 and the expression of downstream genes in the cell model simulating dry macular degeneration.

Example 3: Analysis of therapeutic effect in animal model simulating dry macular degeneration induced by NaIO$_3$ using nucleic acid complex

In this Example, nucleic acid complex combinations for which the effects were verified in Example 2 were selected, and the phenotypes and histological findings thereof were analyzed in an animal model simulating dry macular degeneration using NaIO$_3$. The nucleic acid complex combinations that were used are given in Table 6 below.

[Table 6]

| Nucleic acid complex combination for analysis of therapeutic effect on dry macular degeneration in animal model induced by NaIO$_3$ | |
|---|---|
| Classification | Nucleic acid complex |
| 1 | SEQ ID NOS: 1 and 5 |
| 2 | SEQ ID NOS: 3 and 10 |

Example 3-1: Experimental animal breeding and experimental design

5-week-old male B6 mice were used after acclimatization for 1 week. 35 mg/kg of NaIO$_3$ (Sigma, USA) was intravenously injected thereto in order to induce selective degeneration of and damage to retinal pigment epithelial cells, which are changes that appear in macular degeneration.

After 3 weeks of induction using NaIO$_3$, the experiment was conducted in a manner in which the nucleic acid complexes (1, 2) were used in the form of eye drops at 60 μg / 1 drop once a day for 14 days for a control group (nucleic acid complex 1) and at 60 μg / 1 drop once a day for 14 days for a test drug group (nucleic acid complex 2).

After administration of the nucleic acid complexes (1, 2) for 14 days, fundus imaging was performed using a Micron IV (Phoenix Research Laboratories, USA).

After autopsy and eye extraction, the eye was fixed in 4% paraformaldehyde and embedded in paraffin, and slide sections were made. The slide sections were stained with hematoxylin & eosin (H&E), and retinal tissue was observed using an optical microscope.

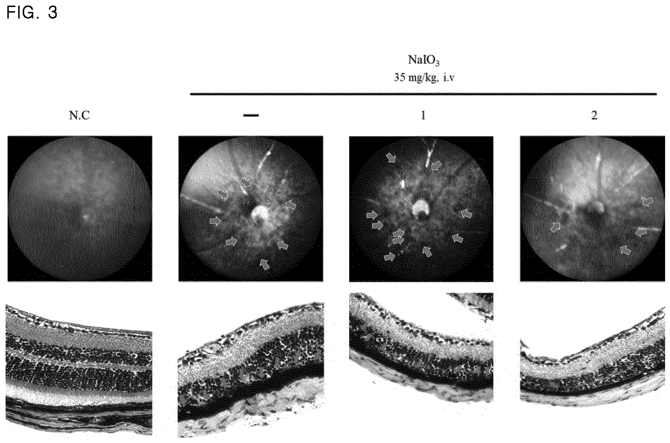

Based on the results thereof, as shown in FIG. 3, the retinal pigment epithelial cells were damaged due to

$NaIO_3$ administration, and warping was observed in the outer nuclear layer, in which the nuclei of photoreceptor cells located directly thereon were dense, and the inner nuclear layer. In the group treated with the nucleic acid complex used in the form of eye drops, it was confirmed that damage to the retinal pigment epithelial cells was inhibited, thereby suppressing the collapse of the outer nuclear layer.

Example 3-2: Effect of 4-week administration of nucleic acid complex (60 $\mu$g) on NLRP3 target gene

Under the experimental conditions of Example 3-1, the nucleic acid complexes (1, 2) were used in the form of eye drops at 60 $\mu$g / 1 drop / 1 day for 28 days in order to evaluate the effect of preventing damage to the retinal pigment epithelium.

After administration of the nucleic acid complexes (1, 2) for 28 days, fundus imaging was performed using a Micron IV (Phoenix Research Laboratories, USA).

After autopsy and eye extraction, the eye was fixed in 4% paraformaldehyde and embedded in paraffin, and slide sections were made. The slide sections were stained with hematoxylin & eosin (H&E), and retinal tissue was observed using an optical microscope.

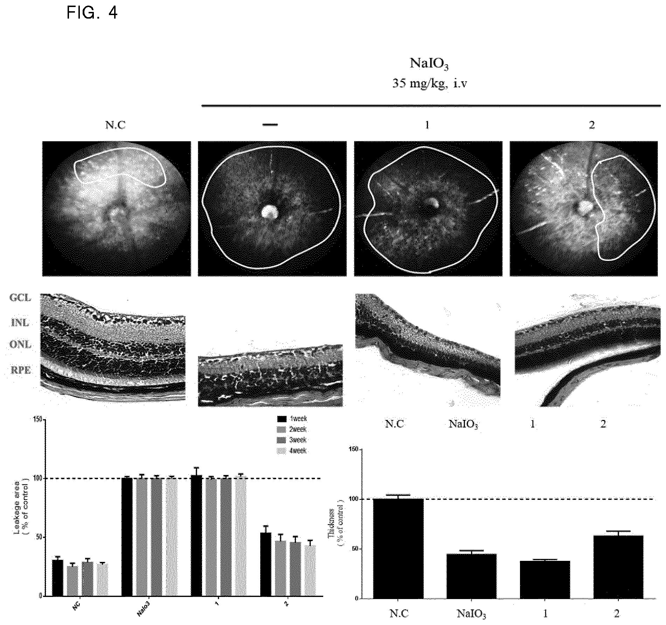

Based on the results thereof, as shown in FIG. 4, the retinal pigment epithelial cells were damaged due to $NaIO_3$ administration, and warping was observed in the outer nuclear layer, in which the nuclei of photoreceptor cells located directly thereon were dense, and the inner nuclear layer. In the group treated with the nucleic acid complex used in the form of eye drops, it was confirmed that damage to the retinal pigment epithelial cells was inhibited, thereby suppressing warping of the outer nuclear layer.

Example 3-3: Analysis of therapeutic effect in animal model induced by $NaIO_3$ using nucleic acid complex

Under the experimental conditions of Example 3-1, the nucleic acid complexes (6, 7) of Table 7 were used in the form of eye drops in order to evaluate the inhibitory effect on damage to the retinal pigment epithelium. The nucleic acid complex No. 6 was administered at 30 $\mu$g / 1 drop / 1 day for 14 days, and the nucleic acid complex No. 7 at 30 $\mu$g / 1 drop / 1 day, the nucleic acid complex No. 7-1 at 10 $\mu$g / 1 drop / 1 day, and the nucleic acid complex No. 7-2 at 5 $\mu$g / 1 drop / 1 day were administered for 14 days, and the inhibitory effect was confirmed. For a positive control, MCC950 (InvivoGen, USA), which is an NLRP3 inhibitor, was orally administered daily for 14 days at a dose of 10 mg/kg.

[Table 7]

| Additional nucleic acid complex combination for analysis of therapeutic effect on dry macular degeneration in animal model induced by $NaIO_3$ | |
| --- | --- |
| Classification | Nucleic acid complex |
| 6 | SEQ ID NOS: 2 and 6 |
| 7 | SEQ ID NOS: 4 and 11 |

After administration of the nucleic acid complexes (6, 7) for 14 days, fundus imaging was performed using a Micron IV (Phoenix Research Laboratories, USA).

After autopsy and eye extraction, the eye was fixed in 4% paraformaldehyde and embedded in paraffin, and slide sections were made. The slide sections were stained with hematoxylin & eosin (H&E), and retinal tissue was observed using an optical microscope.

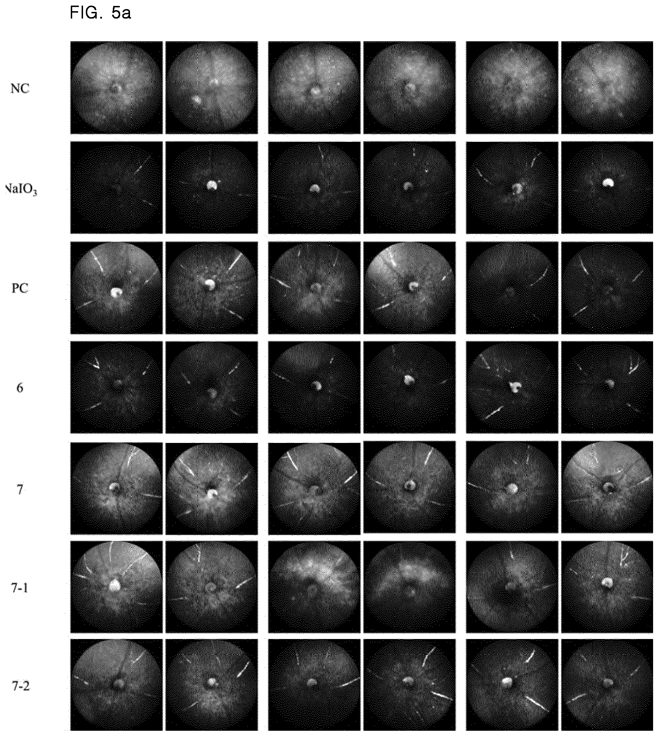

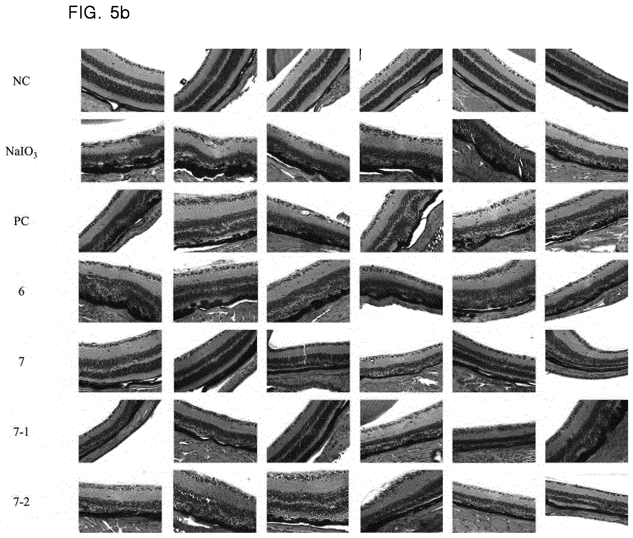

Based on the results thereof, as shown in FIG. 5, it was observed that retinal degeneration was caused by inducing degeneration of retinal pigment epithelial cells through oxidative stress due to $NaIO_3$ administration, and the nucleic acid complex (PNA 7, 30 $\mu$g / 1 drop / 1 day) was confirmed to reduce degeneration of retinal pigment epithelial cells compared to the positive control (PC). In the nucleic acid complex (PNA 7-1: 10 $\mu$g / 1 drop / 1 day, PNA 7-2: 5 $\mu$g / 1 drop / 1 day), an inhibitory effect similar to that of the positive control was confirmed (FIG. 5a).

For the thickness of the outer nuclear layer, in which the nuclei of photoreceptor cells in the retina are dense, as shown in the retinal section images of FIG. 5b, damage to the photoreceptor cells was significantly inhibited in the group treated with the nucleic acid complex (PNA 7) used in the form of eye drops, indicating the therapeutic effect on dry macular degeneration.

Although specific embodiments of the present invention have been disclosed in detail above, it will be obvious to those of ordinary skill in the art that the description is merely of preferable exemplary embodiments, and is not to be construed as limiting the scope of the present invention. Therefore, the substantial scope of the present invention will be defined by the appended claims and equivalents thereof.

[Industrial Applicability]

According to the present invention, a cell-permeable nucleic acid complex in which a bioactive nucleic acid targeting NLRP3 and a carrier peptide nucleic acid are complementarily bound to each other has high cell permeation capability, so it can be administered through various methods, including eye drops or skin application, in addition to direct injection, and is capable of inhibiting expression of NLRP3 and is thus useful for the prevention, amelioration, or treatment of macular degeneration.

[Sequence List Free Text]

An electronic file is attached.

**Claims**

1.  A pharmaceutical composition for preventing, ameliorating, or treating macular degeneration comprising:

    a cell-permeable nucleic acid complex containing a bioactive nucleic acid targeting an NLRP3 gene; and
    a carrier peptide nucleic acid which are complementarily bound to said bioactive nucleic acid, as an active ingredient.

2.  The pharmaceutical composition according to claim 1, wherein the bioactive nucleic acid comprises a sequence represented by a sequence of SEQ ID NO: 4.

3.  The pharmaceutical composition according to claim 1, wherein the carrier peptide nucleic acid is represented by a sequence selected from the group consisting of SEQ ID NOS: 5 to 11.

4.  The pharmaceutical composition according to claim 1, wherein the nucleic acid complex comprises a bioactive nucleic acid comprising a sequence represented by SEQ ID NO: 4 and a carrier peptide nucleic acid comprising a sequence represented by any one sequence selected from the group consisting of SEQ ID NOS: 5 to 11.

5.  The pharmaceutical composition according to claim 1, wherein the bioactive nucleic acid or the carrier peptide nucleic acid comprises a material facilitating endosomal escape that is additionally bound to a 5'-end or a 3'-end of each nucleic acid.

6.  The pharmaceutical composition according to claim 5, wherein the material facilitating endosomal escape is at least one selected from the group consisting of a peptide, lipid nanoparticles, polyplex nanoparticles, polymer nanospheres, inorganic nanoparticles, cationic lipid-based nanoparticles, a cationic polymer, and a pH-sensitive polymer.

7.  The pharmaceutical composition according to claim 6, wherein the peptide is GLFDIIKKIAESF (SEQ ID NO: 12) or histidine (10).

8.  The pharmaceutical composition according to claim 1, wherein the bioactive nucleic acid has an overall negative charge or no charge.

9.  The pharmaceutical composition according to claim 1, wherein the carrier peptide nucleic acid has an overall positive charge.

10. The pharmaceutical composition according to claim 1, wherein the nucleic acid complex has an overall positive charge.

FIG. 1

FIG. 2a

FIG. 2b

FIG. 3

FIG. 4

FIG. 5a

NC

NaIO₃

PC

6

7

7-1

7-2

FIG. 5b

NC

NaIO₃

PC

6

7

7-1

7-2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101963885
- US 8283461 B
- US 8313772 B
- US 8501930 B
- US 4235871 A
- US 4501728 A
- US 4837028 A
- US 5019369 A

### Non-patent literature cited in the description

- **LUCIA CELKOVA et al.** NLRP3 Inflammasome and Pathobiology in AMD. *J. Clin. Med.,* January 2015, vol. 4 (1), 172-192
- **VALERIA TARALLO et al.** *Cell,* 11 May 2012, vol. 149 (4), 847-59
- **KOLE R. et al.** *Nature Rev. Drug Discov.,* 2012, vol. 11, 125-140
- **WILSON C. et al.** *Curr. Opin. Chem. Bio.,* 2006, vol. 10, 607-614
- **COUTO L.B. et al.** *Curr. Opin. Pharmacol.,* 2010, vol. 5, 534-542
- **ZHI D. et al.** *Bioconjug. Chem.,* 2013, vol. 24, 487-519
- **BUYENS K. et al.** *J. Control Release,* 2012, vol. 158, 362-70
- **ROSSI, J. J. et al.** *Gene Ther.,* 2006, vol. 13, 583-584
- **YOUSEFI A. et al.** *J. Control Release,* 2013, vol. 170, 209-18
- **TRABULO S. et al.** *Curr. Pharm. Des.,* 2013, vol. 19, 2895-923
- **D.W. PACK ; A.S. HOFFMAN ; S. PUN ; P.S. STAYTON.** Design and development of polymers for gene delivery. *Nat. Rev. Drug. Discov.,* 2005, vol. 4, 581-593
- **LUCIA CELKOVA.** *Journal of Clinical Medicine,* 2015, vol. 4 (1), 172-192
- **YERRAMOTHU P. et al.** *Eye,* 2018, vol. 32, 491-505
- **MINAKUCHI et al.** *Nucleic Acids Res.,* 2004, vol. 32 (13), e109
- **HANAI et al.** *Ann. NY Acad. Sci.,* 2006, vol. 1082, 9-17
- **KAWATA et al.** *Mol. Cancer Ther.,* 2008, vol. 7 (9), 2904-12
- **SZOKA et al.** *Ann. Rev. Biophys. Bioeng.,* 1980, vol. 9, 467